Europäisches Patentamt

European Patent Office

Office européen des brevets

(11) Publication number: **0 140 606 B1**

# EUROPEAN PATENT SPECIFICATION

(45) Date of publication of patent specification: **15.05.91**

(51) Int. Cl.5: **C12P 1/00, C12N 15/00, C12P 13/22, C12N 5/00, C12N 5/02, //C12R1/19, C12R1/07,C12R1/465, C12R1/22,C12R1/42,C12R1/85**

(21) Application number: **84306842.0**

(22) Date of filing: **08.10.84**

(54) Cells and their use in methods for producing product compounds.

(30) Priority: **07.10.83 US 539981**

(43) Date of publication of application:
**08.05.85 Bulletin 85/19**

(45) Publication of the grant of the patent:
**15.05.91 Bulletin 91/20**

(84) Designated Contracting States:
**AT BE CH DE FR GB IT LI NL SE**

(56) References cited:
**DE-A- 3 128 411**

**THE BIOCHEMICAL JOURNAL, vol. 99, no. 1, 1966, London;H.L.KORNBERG: "The Role and Control of the Glyoxylate Cycle in Escherichia coli", pp. 1-11**

**JOURNAL OF BACTERIOLOGY, vol. 127, no. 1, July 1976, Washington , D.C. USA;T.A. KRULWICH et al.:"Natural Paucity of Anaplerotic Enzymes: Basis for Dependence of Arthobacter pyridinolis On L-Malate for**

**Growth", pp. 179-183**

(73) Proprietor: **BIOTECHNICA INTERNATIONAL, INC.**
**85 Bolton Street**
**Cambridge Massachusetts 02140(US)**

Proprietor: **H.J. HEINZ COMPANY**
**World Headquarters 600 Grant Street**
**Pittsburgh Pennsylvania 15219(US)**

(72) Inventor: **Backman, Keith C.**
**200 Carlisle Road**
**Bedford Massachusetts(US)**

(74) Representative: **Deans, Michael John Percy et al**
**Lloyd Wise, Tregear & CO. Norman House**
**105-109 Strand**
**London WC2R OAE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to producing a desired compound and to cells used in such production.

In conducting fermentation, the cell mass should be adequate to convert raw material into product, but product yields will be reduced if raw material is squandered in producing a cell mass substantially in excess of the amount needed for a reasonable rate of raw material conversion.

Accordingly, it is desirable to control cell mass, for example by controlling nutrients in the cell medium. At the same time, the method used to control cell mass should not adversely affect the ability of the cell structure to produce the desired fermentation product.

According to a first aspect of the present invention, there is provided a method of producing a product compound using cells to convert a raw material into said product compound, said product compound being a compound that is not part of the glycolytic pathway, the method comprising the steps of: selecting as cells to accomplish said conversion cells that: (a) are defective in their ability to replenish tricarboxylic acid cycle (TAC) intermediates from glycolytic pathway products and b) have been engineered to convert, or enhance conversion of, said raw material to said product compound via a biosynthesis pathway that is free from any TAC intermediates; culturing the selected cells in a culture medium; and recovering said product compound from said medium.

As used herein, "glycolytic pathway products" means the products in the Embden-Meyerhof pathway sequence: glucose; glucose-6-phosphate; fructose-6-phosphate; fructose-1,6-diphosphate; dihydroxyacetone-phosphate; glyceraldehyde-3-phosphate; 1,3-diphosphoglycerate; 3-phosphoglycerate; 2-phosphoglycerate; phosphoenolpyruvate (PEP); pyruvate; acetaldehyde; and acetate. The TAC intermediates are succinate, oxalacetate, malate, fumarate, 2-ketoglutarate, isocitrate, and citrate.

In a second and alternative aspect of this invention, that is provided a cell characterised by permanent incapacity to form oxalacetate from glycolytic pathway products, said cell being engineered to enhance or to enable production of a desired product compound via a bioconversion pathway that is free from any tricarboxylic acid cycle (TAC) intermediates, said product compound being a compound that is not part of the glycolytic pathway.

In preferred cells, there is a vector comprising DNA encoding an enzyme that catalyzes a reaction in the said bioconversion pathway and the cells are capable of replacing TAC intermediates solely via the glyoxalate shunt, which comprises reactions catalyzed by isocitrate lyase and malate synthase, and is subject to inhibition.

In preferred arrangements, the cells are just cultured to a desired cell mass under growth medium conditions such that cells can replace TAC intermediates in sufficient amounts to support growth of the cells, and are thereafter cultured under product-production medium conditions such that cell growth is substantially inhibited due to inadequate replacement of TAC intermediates.

In this way we provide a convenient method of controlling the extent of the cell mass. The method is not dependent on limiting any nutrient that is required for effecting the conversion of raw material to end product, and the method channels the cell's metabolism away from growth pathways, thereby increasing the efficiency of conversion of raw material to end product.

Other features and advantages of the invention will be apparent from the following description of the preferred embodiments and from the claims.

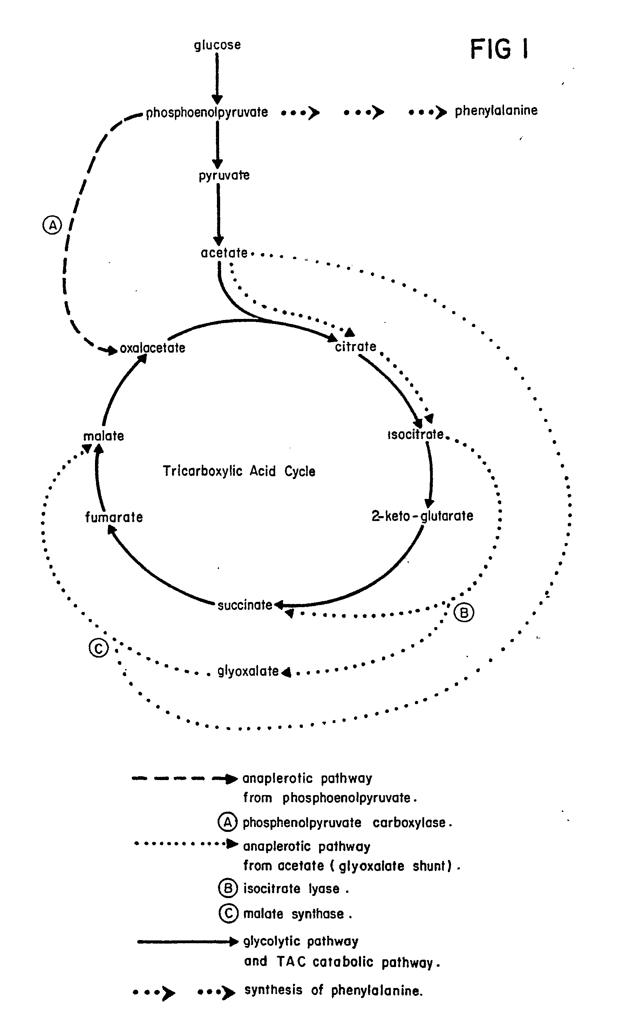

The single Fig 1 of the drawings is a flow diagram showing various bioconversion pathways used in growth and fermentation.

### The Microbial Cells

In the preferred embodiment, the metabolic characteristics of the cells are illustrated in Fig. 1. In Fig. 1, cells derive energy from carbohydrates by degradation through a variety of pathways to PEP, which enters the tricarboxylic acid pathway. As used in this application, "carbohydrates" means glucose, other saccharides, and polysaccharides which the cell can hydrolyze to saccharides. The TAC includes a catabolic route (shown in solid line in Fig. 1), which produces energy using the four-carbon intermediates quasicatalytically: ie, a highly oxidized four-carbon intermediate is fused to a less oxidized two-carbon intermediate; then two relatively highly oxidized one-carbon molecules (carbon dioxide) are severed leaving a less oxidized four-carbon molecule. Useful energy is derived by oxidizing that four-carbon molecule back to the starting point; ie, oxalacetate. The net effect of this catabolic portion of the TAC is production of energy and carbon dioxide with a net loss of acetate. There is no net consumption of the four carbon TAC intermediates.

The TAC has another important function in the cell, however. It provides a pool of biosynthetic precursors for a variety of important cellular components, including amino acids and nucleic acids. Thus, while the four-carbon TAC intermediates are not consumed in the above-described catabolic cycle, these intermediates are siphoned off, particularly during cell growth, to form various cellular components, and a mechanism for replacing these intermediates is needed to support cell growth.

Pathways for replacing TAC intermediates are known as anaplerotic (filling-up) pathways. In one such pathway (dashes in Fig. 1), PEP is converted to oxalacetate via the enzyme phosphoenolpyruvate carboxylase. In another such pathway (dots in Fig. 1), called the glyoxalate shunt of the TAC cycle, citrate is converted (via isocitrate) to succinate and glyoxalate by the enzyme isocitrate lyase. Acetate is combined with glyoxalate by the enzyme malate synthase to yield malate. The net result of the glyoxalate shunt is the replenishing of the TAC by a gain of a four-carbon TAC intermediate at the expense of two two-carbon molecules (acetate).

A cell that has lost its ability to replenish TAC intermediates from phosphoenolpyruvate can nevertheless grow on acetate, because TAC intermediates are replenished via the glyoxylate shunt. However, the glyoxylate shunt is inhibited by products of the glycolytic pathway upstream from acetate. Thus cells which are capable of growth on acetate alone are inhibited from growth by the addition of glucose. [Kornberg (1966) Biochem. J. 99:1-11; Krulwich et al. (1976) J. Bateriol. 127:179-183].

It is possible to develop cell strains whose growth on a non-carbohydrate such as acetate is inhibited by glycolytic pathway substances produced from carbohydrates, and which nevertheless retain their viability and their ability to produce a desired fermentation product in the presence of carbohydrates and/or acetate. As explained below, growth of such cells may be readily controlled, e.g. in a fermentation process. The products thus produced include compounds which: 1) are not produced in the glycolytic pathway; and 2) are produced by a pathway which does not produce a net increase of a TAC intermediate.

The cells may be formed by modifying a parent cell to enable (or enhance the effectiveness of) a pathway for forming those product compounds, and modifying parent cells to impair their ability to form phosphoenolpyruvate carboxylase, and thus their ability to convert PEP to oxalacetate.

Suitable cell lines include those belonging to the following genera: Bacillus, Streptomyces, Escherichia, Klebsiella, Salmonella, and Saccharomyces, and particularly strains derived from wild-type strains capable of converting carbohydrates to TAC intermediates. A particularly preferred species is E. coli, and strains thereof described below which are derived from the K12 strain YMC9, and which lack the ability to synthesize phosphoenolpyruvate carboxylase (ppc), an enzyme necessary for conversion of glucose to TAC intermediates, by virtue of lesions in the ppc gene.

## A. KB260

Strain KB260, a strain which demonstrates control of cell-mass growth, is constructed as follows. Strain YMC9 (ATCC accession No. 33927) is mutagenized with the mud(amp lac)1 random insertional mutator element according to the procedure of Casadaban and Cohen (Casadaben, M.J., and Cohen, S.N. Proceedings of the National Academy of Sciences 76:4530-4533 [1979]). Cells which have been mutated re selected by growth in the presence of ampicillin, since acquisition of the mutator element results in resistance to $\beta$-lactam antibiotics. A large pool of random mutants is selectively enriched for mutants unable to synthesize arginine by growing the pooled mutants in M9 minimal salts supplemented with 2mg/ml glucose with 1 $\mu$g/ml thiamine and 40 $\mu$g/ml L-arginine at 30°C until the culture is growing logarithmically, next collecting and washing the cells by filtration, resuspending the cells in M9 minimal salts with 2mg/ml glucose and 1 $\mu$g/ml thiamine, incubating the cells at 30°C for 1 to 1.5 hours, adding D-cycloserine (final concentration: 0.002M), and collecting the viable cells which remain after lysis of the culture. Among these cells are mutants of YMC9 which cannot grow in the absence of exogenous arginine (or a biosynthetic precursor of arginine). Typical of such mutants is strain KB243 which is capable of growth when the medium is supplemented with arginine, or with either ornithine or citrulline, which are precursors of arginine.

Strain KB243 is plated on agar plates containing M9 salts, 2mg/ml glucose, 1 $\mu$g/ml thiamine, 40 $\mu$g/ml L-arginine, and 0.5mg/ml succinate and incubated at 42°C. This results in the death of most cells on the plate because of lethal functions on the mud(amp lac)1 element. Among the surviving bacteria are deletion mutants which had lost portions of the mud (amp lac) 1 element and surrounding DNA. An example of such a strain is KB244, which has lost genetic information for resistance to $\beta$-lactam antibiotics and is incapable of growth when provided with ornithine or citrulline instead of arginine. This last property identifies the lesions in the arginine biosynthetic pathway as falling in the genes of the argECBH cluster, which is linked to the ppc gene on the E. coli chromosome (Bachmann, B.J. Microbiological Reviews 47:180-230 [1983]).

Strain KB244 is transduced to arginine prototrophy with P1 bacteriophage grown on E. coli strain DL10 (LeMaster, D.M. and Cronan, Jr., J.F. Journal of Biological Chemistry 257:1224-1230 [1982]) which has an unmutated argECBH cluster but carries a mutation in the ppc gene. Isolates are examined for growth on M9 minimal salts plus 2mg/ml glucose and 1 μg/ml thiamine, plus or minus 0.5mg/ml succinate. Strains which acquire a ppc⁻ allele are unable to grow without the succinate. Typical of the ppc⁻ transductants obtained is KB260.

The mutation in the KB260 gene responsible for affecting phosphoenolpyruvate carboxylase (PPC) synthesis is a point mutation, and, therefore, there is the possibility of reversion, resulting in acquisition of the ability to synthesize PPC. Other strains are described below in which the loss of PPC synthesis is attributable to a type of mutation that is less susceptible to reversion.

B. KB280 and KB285

Strains KB280 and KB285, which demonstrate control of cell-mass growth in a fermentation process, are constructed as follows. Strain YMC9 is mutagenized with the mud(amp lac)1 insertional mutator element, as above. A pool of mutated cells is enriched for ppc⁻ mutants by growing them on M9 minimal salts with 2mg/ml glucose, 1 μg/ml thiamine, and 0.5mg/ml succinate at 30°C until the culture is growing logarithmically, collecting and washing the cells by filtration, resuspending the cells in M9 minimal salts plus 2mg/ml glucose, and 1μg/ml thiamine incubating the cells at 30°C for 1 to 1.5 hours, adding D-cycloserine (final concentration: 0.002M) and collecting the viable cells which remain after lysis of the culture. Among such cells are mutants which require exogenous succinate for growth in the presence of glucose. Typical of such strains are KB279 and KB284. Cells of each strain are plated on agar plates containing M9 salts, 2mg/ml glucose, 1 μg/ml thiamine, 0.5mg/ml succinate, and 40 μg/ml L-arginine and incubated at 42°C. Among survivors are bacteria which have lost portions of the mud (amp lac)1 element and surrounding DNA. Such strains are no longer resistant to β -lactam antibiotics, and certain isolates have acquired a requirement for exogenous arginine for growth.

This property confirms that the lesions in KB279 and KB284 are in fact in the ppc gene. Isolates KB280 (from KB279) and KB285 (from KB284) are saved. Each has lost its progenitor's resistance to β -lactam antibiotics, but neither has acquired a defect in arginine biosynthesis. KB280 and KB285 have been deposited with the American Type Culture Collection and have accession numbers ATCC 39461 and ATCC 39463, respectively.

Controlling Cell-Mass.

The strains described above are cultured to a predetermined cell mass, and then further cell mass growth is readily controlled. Specifically, the strain is cultivated on a carbohydrate-free medium. If carbohydrates are present initially, they will be metabolized to inhibiting compounds which will stop growth. Eventually, the carbohydrates and glycolytic intermediates may be depleted by various metabolic pathways, and growth can begin. Once desired cell mass is achieved (e.g. after about eight hours) a carbohydrate is added to inhibit continued cell growth by inhibiting the non-glucose-to-TAC pathway, thus depriving the cell of its only source of TAC cycle intermediates needed for cell growth. The amount of carbohydrate added will depend largely on whether the carbohydrate serves solely as a generator of inhibiting substance, or whether it also serves as the raw material for production of the desired compound. About 2 to 4 mg/ml of glucose is satisfactory to inhibit cell growth. More glucose is added (either at the outset of the fermentation phase or continuously during that phase) when glucose serves as the raw material for fermentation in order to maintain sufficient raw material levels to drive fermentation pathway sections.

As specifically related to the above-described strains of E. coli, strain KB260 is inoculated into a culture containing 20 ml of M9 salts, 4mg/ml sodium acetate, 1 μg/ml thiamine and grown at 37°C. Culture turbidity is monitored with a Klett-Summerson Colorimeter. When the turbidity reaches 40-50 Klett units (green filter), glucose (final concentration: 4mg/ml) is added to the KB260 culture, and culture turbidity of all cultures is monitored for another nineteen hours. The number of viable cells in the KB260 culture is also determined four hours and nineteen hours after the addition of glucose.

The growth rate of KB260 is like that of its parent YMC9 when grown entirely on acetate, but KB260 and YMC9 respond dramatically differently to the addition of glucose. YMC9 increases its growth rate and reaches a higher saturation density with glucose than either YMC9 or KB260 on acetate alone. KB260 in contrast, ceases accumulation of cell mass shortly after the addition of glucose, and fails to accumulate new cell mass over intermediate to long times. Unlike many other schemes in which an added substance halts cell growth, however, the addition of glucose to the KB260 culture does not significantly impair the viability of the cells.

Producing The Desired Product.

Depending on the desired product and the capability of the parent cells, strains may require modification, such as by the addition of a plasmid with DNA coding for an enzyme catalyzing a reaction in the product fermentation pathway. For example, to produce L-phenylalanine (a component of a non-sugar sweetener useful in a wide variety of food products) the strains described above are transformed with a plasmid pKB663 described below.

A fragment of DNA carrying the pheA gene of E. coli but not its associated promoter, operator, leader peptide, or attenuator is prepared from plasmid pKB45 (Zurawski, G., et al. Proceedings of the National Academy of Sciences 75:4271-4274 [1978]) by digestion with endonucleases StuI and BglII. Plasmid pKB430 is a derivative of pBR322 which carries a lactose operon promoter-operator abutting an endonuclease PvuII cleavage site. The pheA containing DNA fragment is cloned by standard techniques (Bolivar, F. and Backman, K. Methods in Enzymology, Vol. 68 [1980]) in pKB430 between the PvuII and BamHI cleavage sites, yielding pKB663. On pKB663, expression of pheA is directed by the lac promoter and is not regulated in response to accumulation of L-phenylalanine, as is the pheA gene when associated with its normal regulatory elements. pKB663 also carries a gene which determines resistance to $\beta$-lactam antibiotics such as ampicillin. Plasmid pKB663 is available from YMC9/pKB663 which is deposited with the American Type Culture Collection and has accession number ATCC 39462.

Strains YMC9, KB280, and KB285 are transformed with pKB663 (see Bolivar, F. and Backman, K. Methods in Enzymology, Vol. 68 [1980]) yielding YMC9/pKB663, KB280/pKB663, and KB285/pKB663. Due to their tendency to shed their plasmids, these strains are maintained on agar plates containing M9 salts, 2mg/ml glucose, 0.5mg/ml succinate, 1 $\mu$g/ml thiamine, and 100 $\mu$g/ml ampicillin.

Cultures of YMC9/pKB663, KB280/pKB663, and KB285/pKB663 are grown at 37°C in 20ml of M9 salts, 4mg/ml sodium acetate, 1$\mu$g/ml thiamine to a density of either 45 to 60 or 130 to 150 Klett units (green filter), at which time the culture is collected by filtration, the cells are resuspended in an equal volume of fresh medium, and glucose is added to a final concentration of 4mg/ml. The pattern of cell growth thereafter resembles YMC9 (described above) (for YMC9/pKB663) or KB260 (also described above) (for KB280/pKB663 and KB285/pKB663) following glucose addition. Incubation is continued at 37°C for 10 to 15 hours, at which point culture density, cell viability, and L-phenylalanine content of the medium are determined.

Additional information concerning the method of modifying YMC9 and the method of culturing the modified cells to produce L-phenylalanine is described in our co-pending European Patent Application No. 84306841.2 (Publication No. EPO145156), the disclosure of which is hereby incorporated by reference.

Other arrangements are possible within the scope of this invention. For example, the cells used may be microbial cells or cell tissue from multi-celled organisms.

## Claims

1. A method of producing a product compound using cells to convert a raw material into said product compound, said product compound being a compound that is not part of the glycolytic pathway, the method comprising the steps of: selecting as cells to accomplish said conversion cells that: (a) are defective in their ability to replenish tricarboxylic acid cycle (TAC) intermediates from glycolytic pathway products and b) have been engineered to convert, or enhance conversion of, said raw material to said product compound via a biosynthesis pathway that is free from any TAC intermediates; culturing the selected cells in a culture medium; and recovering said product compound from said medium.

2. A method according to Claim 1, wherein said cells are ppc-.

3. A method according to Claim 1, wherein said cells are capable of replacing TAC intermediates solely via the glyoxalate shunt, which is subject to inhibition.

4. A method according to any preceding Claim, wherein said product compound is produced under conditions effectively preventing replacement of TAC intermediates.

5. A method according to any preceding Claim, comprising
   (a) first culturing said cells to a desired cell mass under growth medium conditions such that said cells can replace TAC intermediates in sufficient amounts to support growth of said cells, and
   (b) thereafter culturing said cells under product-production medium conditions such that cell growth is substantially inhibited due to inadequate replacement of TAC intermediates.

6. A method according to any preceding Claim, wherein said cells are permanently incapable of forming oxalacetate from glycolytic pathway products, and are capable of converting acetate or acetic acid to succinate or malate via the glyoxalate shunt, in the absence of an inhibiting substance.

7. A method according to Claim 6, wherein said inhibiting substance is glucose or a compound derived from glucose by glycolysis.

8. A method according to each of Claims 5 to 7, wherein said growth medium lacks TAC-replacement-inhibiting levels of glucose.

9. A method according to any preceding Claim, wherein said product compound is an aromatic amino acid.

10. A method according to Claim 9, wherein said product compound is phenylalanine.

11. A method according to any preceding Claim, wherein said cells are microbial cells, preferably microbial cells selected from any of the genus Bacillus, Streptomyces, Escherichia, Klebsiella, Salmonella, and Saccharomyces.

12. A method according to Claim 11, wherein said microbial cells are members of the species Escherichia coli.

13. A method according to Claim 12, wherein said microbial cells are E. coli K12 strain KB280, E. coli K12 strain KB285, or E. coli K12 strain YMC9, which have been engineered as defined in Claim 1.

14. A method according to Claim 1 or Claim 7, wherein said cells comprise a cloning vector to enhance the ability of said cells to produce said product compound.

15. A method according to Claim 14, wherein said cloning vector comprises DNA coding for an enzyme that catalyzes a step in said synthesis pathway.

16. A method according to Claim 14, wherein said cells are enhanced in their ability to produce phenylalanine by the inclusion of a deregulated pheA gene.

17. A cell characterised by permanent incapacity to form oxalacetate from glycolytic pathway products, said cell being engineered to enhance or to enable production of a desired product compound via a bioconversion pathway that is free from any tricarboxylic acid cycle (TAC) intermediates, said product compound being a compound that is not part of the glycolytic pathway.

18. A cell according to Claim 17, wherein said cell is capable of replacing TAC intermediates solely via the glyoxalate shunt, which is subject to inhibition.

19. A cell according to Claim 17 or 18, wherein said product compound is an aromatic amino acid.

20. A cell according to Claim 19, wherein said cell is engineered to include a deregulated pheA gene.

21. A cell according to Claim 17, wherein said cell comprises a vector comprising DNA encoding an enzyme that catalyzes a reaction in said bioconversion pathway.

22. A cell according to Claim 17, wherein said cell is a member of genus Bacillus, Steptomuyces, Escherichia, Klebsiella, Salmonella, or Saccharomyces.

23. A cell according to Claim 22, wherein said cell is E. coli K12 strain KB280 or E. coli strain KB285, which has been engineered as defined in Claim 17.

24. A cell according to Claim 23, wherein said cell is deposited as ATCC Accession No. 39461 or 39463.

**Revendications**

1. Procédé de production d'un composé formant produit au moyen de cellules capables de transformer une matière première en ledit composé formant produit, ledit composé formant produit étant un composé qui ne participe pas à la voie glycolytique, le procédé comprenant les étapes de choix, comme cellules destinées à accomplir ladite conversion, de cellules qui : (a) sont déficientes concernant leur aptitude à reformer les intermédiaires du cycle de Krebs à partir de produits de la voie glycolytique et (b) qui ont été transformées pour convertir ladite matière première ou améliorer la conversion de ladite matière première en ledit composé formant produit par l'intermédiaire d'une voie de biosynthèse qui est exempte de tout intermédiaire du cycle de Krebs, la culture des cellules choisies dans un milieu de culture et la récupération dudit composé formant produit à partir dudit milieu.

2. Procédé selon la revendication 1, dans lequel lesdites cellules sont des cellules ppc-.

3. Procédé selon la revendication 1, dans lequel lesdites cellules sont capables de remplacer les intermédiaires du cycle de Krebs uniquement par l'intermédiaire du court-circuit au glyoxalate, qui est soumis à une inhibition.

4. Procédé selon l'une quelconque des revendications précédentes, dans lequel ledit composé formant produit est produit dans des conditions empêchant efficacement le remplacement des intermédiaires du cycle de Krebs.

5. Procédé selon l'une quelconque des revendications précédentes, comprenant
    (a) tout d'abord la culture desdites cellules pour obtenir une masse cellulaire souhaitée dans des conditions de milieu de culture telles que lesdites cellules peuvent remplacer les intermédiaires du cycle de Krebs en des quantités suffisantes pour soutenir la croissance desdites cellules, et
    (b) puis la culture desdites cellules dans des conditions de milieu de production de produit telles que la croissance des cellules est sensiblement inhibée du fait du remplacement inadéquat des intermédiaires du cycle de Krebs.

6. Procédé selon l'une quelconque des revendications précédentes, dans lequel lesdites cellules sont en permanence incapables de former de l'oxaloacétate à partir des produits de la voie glycolytique, et sont capables de convertir l'acétate ou l'acide acétique en succinate ou en malate par l'intermédiaire du court-circuit au glyoxalate, en l'absence d'une substance inhibitrice.

7. Procédé selon la revendication 6, dans lequel ladite substance inhibitrice est le glucose ou un composé dérivé du glucose par glycolyse.

8. Procédé selon chacune des revendications 5 à 7, dans lequel ledit milieu de culture est exempt de niveaux de glucose inhibant le remplacement du cycle de Krebs.

9. Procédé selon l'une quelconque des revendications précédentes, dans lequel ledit composé formant produit est un acide aminé aromatique.

10. Procédé selon la revendication 9, dans lequel ledit composé formant produit est la phénylalanine.

11. Procédé selon l'une quelconque des revendications précédentes, dans lequel lesdites cellules sont des cellules microbiennes, de préférence des cellules microbiennes choisies parmi l'un quelconque des genres Bacillus, Streptomyces, Escherichia, Klebsiella, Salmonella et Saccharomyces.

12. Procédé selon la revendication 11, dans lequel lesdites cellules microbiennes sont des membres de l'espèce Escherichia coli.

13. Procédé selon la revendication 12, dans lequel lesdites cellules microbiennes sont la souche KB280 de E. coli K12, la souche KB285 de E. coli K12 ou la souche YMC9 de E. coli K12, qui ont été modifiées selon la revendication 1.

14. Procédé selon la revendication 1 ou la revendication 7, dans lequel lesdites cellules comprennent un vecteur de clonage pour augmenter l'aptitude desdites cellules à produire ledit composé formant produit.

15. Procédé selon la revendication 14, dans lequel ledit vecteur de clonage comprend un ADN codant pour un enzyme qui catalyse une étape de ladite voie de synthèse.

16. Procédé selon la revendication 14, dans lequel l'aptitude desdites cellules à produire la phénylalanine est augmentée par inclusion d'un gène pheA dérégulé.

17. Cellule, caractérisée par une incapacité permanente à former de l'oxaloacétate à partir des produits de la voie glycolytique, ladite cellule étant modifiée pour augmenter ou pour permettre la production d'un composé formant produit souhaité par l'intermédiaire d'une voie de bioconversion qui est exempte de tout intermédiaire du cycle de Krebs, ledit composé formant produit étant un composé qui ne prend pas part à la voie glycolytique.

18. Cellule selon la revendication 17, dans laquelle ladite cellule est capable de remplacer les intermédiaires du cycle de Krebs uniquement par l'intermédiaire du court-circuit au glyoxalate, qui est soumis à une inhibition.

19. Cellule selon la revendication 17 ou 18, dans laquelle ledit composé formant produit est un acide aminé aromatique.

20. Cellule selon la revendication 19, dans laquelle ladite cellule est modifiée pour inclure un gène pheA dérégulé.

21. Cellule selon la revendication 17, dans laquelle ladite cellule comprend un vecteur comprenant un ADN codant pour un enzyme qui catalyse une réaction dans ladite voie de bioconversion.

22. Cellule selon la revendication 17, dans laquelle ladite cellule est un membre des genres Bacillus, Streptomyces, Escherichia, Klebsiella, Salmonella ou Saccharomyces.

23. Cellule selon la revendication 22, dans laquelle ladite cellule est la souche KB280 de E. coli K12 ou la souche KB285 de E. coli, qui a été modifiée selon la revendication 17.

24. Cellule selon la revendication 23, dans laquelle ladite cellule est déposée à l'ATCC sous le n° 39461 ou 39463.

**Ansprüche**

1. Verfahren zur Herstellung einer Produktverbindung unter Verwendung von Zellen, um ein Rohmaterial in die genannte Produktverbindung umzuwandeln, wobei die genannte Produktverbindung eine Verbindung ist, die nicht Teil des Glykolyse-Wegs ist das Verfahren umfassend die Schritte: Auswahl als Zellen, um die genannte Umwandlung zu erzielen, von Zellen, die (a) nicht die Fähigkeit besitzen, Tricarbonsäurezyklus-(TAC)-bzw. Zitronensäurezyklus-Zwischenverbindungen aus Glykolyse-Wegprodukten zu ergänzen und (b) Zellen, die manipuliert sind, um das genannte Rohmaterial in die genannte Produktverbindung umzuwandeln oder dessen Umwandlung in diese über einen Biosyntheseweg zu fördern, der frei von TAC-Zwischenverbindungen ist; Kultivieren der ausgewählten Zellen in einem Kulturmedium; und Gewinnen der genannten Produktverbindung aus dem genannten Medium.

2. Verfahren nach Anspruch 1, worin die genannten Zellen ppc⁻ sind.

3. Verfahren nach Anspruch 1, worin die genannten Zellen fähig sind, TAC-Zwischenverbindungen ausschließlich über den Glyoxalat-Nebenweg zu ersetzen, der der Hemmung unterworfen ist.

4. Verfahren nach einem der vorhergehenden Ansprüche, worin die genannte Produktverbindung unter Bedingungen hergestellt wird, die wirksam das Ersetzen von TAC-Zwischenverbindungen verhindern.

5. Verfahren nach einem der vorhergehenden Ansprüche, umfassend
(a) zuerst das Kultivieren der genannten Zellen zu einer gewünschten Zellenmasse unter Wachstumsmedienbedingungen auf solche Weise, daß die genannten Zellen TAC-Zwischenverbindungen in ausreichenden Mengen ersetzen können, um das Wachstum der genannten Zellen zu unterstützen, und
(b) darauffolgendes Kultivieren der genannten Zellen unter produktproduzierenden Medienbedingungen auf solche Weise, daß das Zellenwachstum aufgrund des unzulänglichen Ersetzens von TAC-Zwischenverbindungen wesentlich gehemmt wird.

6. Verfahren nach einem der vorhergehenden Ansprüche, worin die genannten Zellen permanent unfähig sind, Oxalacetat aus Glykolyse-Wegprodukten zu bilden und fähig sind, Acetat oder Essigsäure in Succinat oder Malat über den Glyoxalat-Nebenweg in Abwesenheit einer hemmenden Substanz umzuwandeln.

7. Verfahren nach Anspruch 6, worin die genannte hemmende Substanz Glucose oder eine von Glucose durch Glykolyse abgeleitete Verbindung ist.

8. Verfahren nach einem der Ansprüche 5 bis 7, worin das genannte Wachstumsmedium nicht genügend Glucose enthält, um den Ersatz von TAC zu hemmen.

9. Verfahren nach einem der vorhergehenden Ansprüche, worin die genannte Produktverbindung eine aromatische Aminosäure ist.

10. Verfahren nach Anspruch 9, worin die genannte Produktverbindung Phenylalanin ist.

11. Verfahren nach einem der vorhergehenden Ansprüche, worin die genannten Zellen Mikrobenzellen, vorzugsweise Mikrobenzellen sind, die aus einer der Gattungen Bacillus, Streptomyces, Escherichia, Klebsiella, Salmonella und Saccharomyces gewählt sind.

12. Verfahren nach Anspruch 11, worin die genannten Mikrobenzellen Mitglieder der Gattung Escherichia coli sind.

13. Verfahren nach Anspruch 12, worin die genannten Mikrobenzellen E.coli K12 Stamm KB280, E.coli K12 Stamm KB285 oder E.coli K12 Stamm YMC9 sind, die wie in Anspruch 1 definiert manipuliert sind.

14. Verfahren nach Anspruch 1 oder Anspruch 7, worin die genannten Zellen einen Klonierungsvektor umfassen, um die Fähigkeit der genannten Zellen zur Produktion der genannten Produktverbindung zu verstärken.

15. Verfahren nach Anspruch 14, worin der genannte Klonierungsvektor DNA umfaßt, die für ein Enzym kodiert, das einen Schritt im genannten Syntheseweg katalysiert.

16. Verfahren nach Anspruch 14, worin die genannten Zellen durch die Aufnahme eines deregulierten PheA Gens in ihrer Fähigkeit verstärkt werden, Phenylalanin zu produzieren.

17. Zelle, gekennzeichnet durch die permanente Unfähigkeit, Oxalacetat aus Glykolyse-Wegprodukten zu bilden, wobei die genannte Zelle manipuliert ist, um die Produktion einer gewünschten Produktverbindung über einen biologischen Umwandlungsweg zu verstärken oder zu ermöglichen, der frei von Tricarbonsäurecyclus(TAC)-Zwischenverbindungen ist und die genannte Produktverbindung eine Verbindung ist, die nicht Teil des Glykolysewegs ist.

18. Zelle nach Anspruch 17, worin die genannte Zelle fähig ist, TAC-Zwischenverbindungen ausschließlich über den Glyoxalat-Nebenweg zu ersetzen, der der Hemmung unterworfen ist.

19. Zelle nach Anspruch 17 oder 18, worin die genannte Produktverbindung eine aromatische Aminosäure ist.

20. Zelle nach Anspruch 19, worin die genannte Zelle manupuliert ist, um ein dereguliertes PheA Gen zu enthalten.

21. Zelle nach Anspruch 17, worin die genannte Zelle einen Vektor enthält, der DNA enthält, die ein Enzym kodiert, das eine Reaktion im genannten biologischen Umwandlungsweg katalysiert.

22. Zelle nach Anspruch 17, worin die genannte Zelle ein Mitglied der Gattung Bacillus, Streptomyces, Escherichia, Klebsiella, Salmonella oder Saccharomyces ist.

23. Zelle nach Anspruch 22, worin die genannte Zelle E.coli K12 Stamm KB280 oder E.coli Stamm KB285 ist, die wie in Anspruch 17 definiert manipuliert ist.

24. Zelle nach Anspruch 23, worin die genannte Zelle unter der ATCC-Akzessionsnummer 39461 oder 39463 hinterlegt ist.

FIG I

anaplerotic pathway
from phosphoenolpyruvate.

(A) phosphenolpyruvate carboxylase.

anaplerotic pathway
from acetate ( glyoxalate shunt).

(B) isocitrate lyase .

(C) malate synthase .

glycolytic pathway
and TAC catabolic pathway.

synthesis of phenylalanine.